Europäisches Patentamt

European Patent Office

Office européen des brevets

(19)

(11) Publication number: **0 018 097**
**B1**

(12) **EUROPEAN PATENT SPECIFICATION**

(45) Date of publication of patent specification: **25.07.84**

(21) Application number: **80300897.8**

(22) Date of filing: **21.03.80**

(51) Int. Cl.³: **A 61 M 31/00,**
**A 61 K 9/02, A 61 K 9/22**

(54) Medicated device for controlled drug release.

(30) Priority: **23.03.79 US 23125**
**23.03.79 US 23126**

(43) Date of publication of application:
**29.10.80 Bulletin 80/22**

(45) Publication of the grant of the patent:
**25.07.84 Bulletin 84/30**

(84) Designated Contracting States:
**BE DE FR GB IT NL**

(56) References cited:
**GB - A - 1 480 615**
**US - A - 3 850 160**
**US - A - 3 854 480**
**US - A - 3 938 515**
**US - A - 3 993 073**
**US - A - 4 005 221**
**US - A - 4 043 339**

(73) Proprietor: **THE UPJOHN COMPANY**
**301 Henrietta Street**
**Kalamazoo, Michigan 49001 (US)**

(73) Proprietor: **Baker, Richard William**
**64485 Redmond-Bend Highway**
**Bend, Oregon (US)**

(73) Proprietor: **Ayres, James Walter**
**2420 Northwest 11th Street**
**Corvalis, Oregon (US)**

(72) Inventor: **Roseman, Theodore Jonas**
**5313 Rugby Street**
**Portage, Michigan (US)**
Inventor: **Carpenter, Osmer Sidney**
**15220 Barton Lake Drive**
**Vicksburg Michigan (US)**
Inventor: **Baker, Richard William**
**64485 Redmond-Bend Highway**
**Bend Oregon (US)**
Inventor: **Ayres, James Walter**
**2420 Northwest 11th Street**
**Corvalis Oregon (US)**

(74) Representative: **Perry, Robert Edward et al,**
**GILL JENNINGS & EVERY 53-64 Chancery Lane**
**London WC2A 1HN (GB)**

## Description

The present invention relates to a medicated device for controlled drug release, and particularly for the single delivery of an pharmaceutical agent for the induction of a desired therapeutic effect in a mannal. The devices are adapted for vaginal or rectal administration.

The advantages of a method involving administering pharmacological agents vaginally or rectally are well known in the art. A few of these advantages are:

(1) Agents which are wholly or partially destroyed or inactivated by low gastric pH or enzymatic degradation in the gastrointestinal tract may by administered without · exposure to this destructive environment;

(2) Agents which are gastric irritants may be administered without causing such irritation;

(3) Agents which are administered by this method avoid the so-called first pass deactivation by the liver which inactivates may drugs following oral administration;

(4) This method is convenient for administration of drugs to adult or pediatric patients who may be unable or unwilling to swallow medication; and

(5) This method of administration is effective for treatment of patients who have histories of vomiting or nausea.

Despite these recognized advantages, there are two principal reasons why vaginal or rectal delivery of drugs is rather infrequently used. These reasons are: (1) poor patient acceptance of the present means for vaginal or rectal administration, e.g., primarily suppositories or pessaries; and (2) uncontrolled, variable delivery rates for the drugs released by the devices. For this latter reason an attending physician must often substantially increase the oral dose otherwise employed or acceptable serum levels when the suppository form of the drug is administered will not be achieved. This is an unacceptable approach for administration of a drug which has a limited therapeutic index, i.e., where side effects accompany closely the desired therapeutic effects.

An ideal vaginal or rectal drug delivery device should meet all the following requirements:

(1) The device should deliver the bulk of its drug content within a predetermined specified time period. Typically, this time period should be 12—24 hours for devices placed in the rectum and 2—10 days for vaginal devices. The device should not contain a large residue of drug following this predetermined time period, since there may be no assurance that the patient will remove the device at the prescribed time, thereby avoiding an overdose.

(2) The rate of delivery of the drug should be rate-controlled and relatively constant during the period of drug release.

(3) The rate of delivery of the drug should be lower than the rate at which the drug can be absorbed by the mucosal tissues lining the body cavity into which the device is inserted. The drug delivery rate is dependent upon the delivery system; however, the absorption rate of a drug is dependent upon physical properties of the particular drug, and these rates differ greatly among various drugs. Few of the drugs which are currently prescribed by physicians are capable of being absorbed by mucosal tissue at a rate greater than 5 $mg/cm^2/day$.

Devices or delivery systems which are currently available do not simultaneously meet all of these requirements.

Known devices which provide rate-controlled drug release comprise a drug reservoir within a drug-permeable or otherwise drug-porous barrier. For example, US—A—327996 describes a drug encapsulated in polymeric walls. For prolonged release, US—A—3948254 describes a reservoir of a solid drug carrier; US—A—3926188 describes a poorly water-soluble crystalline drug in a polymeric core lamina interposed between release rate-controlling laminae; US—A—3710795 describes a polymeric matrix, with drug dispersed therethrough, stressed by an elastic, rate-controlling membrane; US—A—3903880 describes a drug matrix reservoir enclosed in a vinylene-ethyl acetate copolymer barrier; US—A—3938515 describes a drug with a carrier enclosed in a polymeric membrane containing a permeability-modifying polymer such as a polyester; US—A—3911911 describes capsules containing a progestational agent, with permeable walls of a silicone elastomer; and US—A—3854480 describes drug particles dispersed through a polymeric matrix surrounded by a drug-permeable membrane.

Again, for controlled release, US—A—3921636 describes a polymeric matrix containing drug reservoirs and US—A—3978203 describes drug-containing biodegradable and metabolisable polymeric matrices, but neither includes a rate-controlling membrane. US—A—3975350 discloses hydrophilic polyurethane drug matrices.

Drug-containing rectal or vaginal suppositories release drug as they melt down, in situ, at an uncontrolled rate subject to conditions in the body cavity.

US—A—3915898, disclosing a progestational compound-impregnated sponge, is an example of the many known vaginal devices which supply prostaglandins or progestational agents, but does not provide controlled release. US—A—3902493 and GB—A—1480615 disclose catamenial tampons having a drug-bearing film coating, and are examples of medicated vaginal tampons.

Examples of controlled release devices are disclosed in US—A—3920805, where the outer surface of a silastic ring has a prostaglandin dispersed therethrough, and US—A—4043339, where a prostaglandin is embedded in a disc-like silastic elastomer.

There are known ophthalmic devices which provide time-independent controlled release of drug. For example, US—A—3641237 discloses a laminate of a drug-bearing film layer encapsulated by a water-soluble polymeric matrix in film form. US—A—3630220 discloses a release rate-controlling drug reservoir within an ocular tissue-compatible hydrophilic polymeric membrane.

A medicated device according to the present invention, adapted for use as a rectal or vaginal insert, has a surface area (A) of 10—50 $cm^2$, which comprises an inert support, an inner flexible polymeric membrane of substantially uniform thickness ($T_1$) laminated to the support and an outer flexible polymeric membrane of substantially uniform thickness ($T_2$) laminated to the inner membrane, in which the inner membrane contains, dissolved therein, an amount (x) of a systemically active pharmaceutical, characterised in that the device can release an amount (y) of the pharmaceutical at a constant rate of 1 to 1000 $\mu$g/hr over a time (t) no more than 72 hours, when in contact with rectal or vaginal tissue having a non-limiting absorption rate, with respect to the release rate, for the pharmaceutical, and

$$T_2 y \simeq D_2 S_2 At$$
$$2(x-y) \simeq 2AS_1 T_1 + AT_2 S_2$$
$$T_2 S_2 D_1 > 10T_1 S_1 D_2$$
$$y \geqslant 0.58x$$

wherein $D_1$ and $D_2$ are the respective diffusion coefficients of the inner and outer membranes with respect to the pharmaceutical, and $S_1$ and $S_2$ are the respective solubilities of the pharmaceutical in the inner and outer membranes.

Devices of the invention are particularly adapted for the rectal or vaginal administration of a systemically active pharmaceutical (hereinafter sometimes "SAP"). The SAP is preferably lipophilic and, most preferably, a lipophilic anti-leuteal/oxytocic prostaglandin.

Prostaglandins for use in the invention may be naturally-occurring, i.e. biosynthetic derivatives of unsaturated fatty acids, or chemical and pharmacological analogues, having at least 10% the potency of $PGE_2$ or $PGF_{2\alpha}$ in standard laboratory animal anti-luteal or oxytocic tests. Tests for anti-luteal potency are designed to measure luteolytic activity or the ability to cause regression of the corpus luteum; US—A—3852465 describes one such procedure, using the Golden Hamster. Oxytocic potency is assessed on the pregnant mammalian myometrium. Tests on the laboratory animal of choice, the Rhesus monkey (Macaca mulatta), designed to measure the amplitude and frequency of uterine contractions, are widely known; see Kirton et al., New York Academy of Science 180:455 (1971); Fuchs et al., New York Academy of Science 180:531 (1971); and Kirton et al., Prostaglandins 1:319 (1972).

Naturally-occurring prostaglandins include the PGA, PGB, PGC, PGD, $PGF_{2\alpha}$ and $PGF_\beta$ compounds, thromboxanes and prostacyclins. Among the prostaglandins preferred for use in the invention, the most preferred is 15-methyl-$PGF_{2\alpha}$, methyl ester.

SAP compounds, other than prostaglandins, which can be used in the invention (with examples of their utility) include aminophylline and theophylline (to relieve asthma), prochlorperazine and chloropromazine (for the relief of nausea and vomiting and as a tranquilliser), chloral hydrate (for sedative and hypnotic effects), oxymorphone HCl (for narcotic analgesia), belladonna and opium (for analgesic and anti-spasmodic effects), ergotamine tartrate (for the relief of migraine syndrome) and aspirin and other non-steroidal anti-inflammatory compounds (for analgesic, anti-pyretic and anti-rheumatic activity).

The SAP is preferably lipophilic, since this allows ready absorption into the rectal or vaginal epithlial tissues. A simple and convenient technique for assessing lipophilicity is the determination of the n-octanol:water partition coefficient; the SAP is placed in an equilibrated mixture of n-octanol and water, the mixture is shaken until equilibrium and the relative SAP concentrations in the n-octanol and water layers are measured. The procedure is advantageously carried out at ambient temperature (preferably about 25°C) and the quantity of SAP selected is less than the quantity soluble in the aqueous layer. The larger the ratio (concentration n-octanol:concentration in water), the greater the lipophilicity. For example, a natural prostaglandin or an analogue thereof is deemed highly lipophilic provided the given ratio (or partition coefficient) is about equal to, or greater than, that of prostaglandin $F_{2\alpha}$ in free acid form. For numerous pharmaceuticals, lipophilicity may be modified by derivatisation, e.g. a carboxylic acid may be converted to an ester form. For example, prostaglandin $F_{2\alpha}$, methyl ester, is significantly more lipophilic than prostaglandin $F_{2\alpha}$ in its free acid form.

Devices of the invention may be used in the treatment of domestic animals, other mammals and, preferably, humans. It is particularly preferred that the SAP is a prostaglandin used to accomplish a discrete event in the mamalian reproductive cycle, i.e. those physiological events which can be induced by prostaglandin administration of about 72 hours, or less, preferably no more than 24 hours. Such discrete events in the mammalian reproductive cycle include regression of the corpus luteum in estrus-cycling animals, abortion, labour induction, hydatidiform mole removal, uterine evacuation following fetal death *in utero,* cervical dilatation (e.g. preliminary to a dilatation and curretage), and treatment of purulent genital tract diseases of domestic animals (e.g. pyrometra). For each of these various indications, the necessary dosage of a prostaglandin and its required duration of administration upon vaginal or rectal administration can be readily assessed, e.g. by determination of the relative amounts of the prostaglandin required to stimulate the uterus of the pregnant Rhesus monkey upon vaginal administration. A SAP other than a prostaglandin can be selected similarly, taking into account the desired duration of treatment and the known therapeutic potential of the SAP.

Once a particular SAP and the therupeutic objective for treatment have been selected, the desired release rate from the device and therapeutic duration of treatment (t) are selected. While the precise and optimal (i.e. acceptable therapeutic effect with minimisation of side-effects) release rate will vary depending upon the particular mammal and the precise therapeutic duration of treatment, by up to 50% from mammal to mammal, these values are nonetheless predetermined based upon experience and the known pharmacological actions of the particular SAP. For example, a prostaglandin used for induction of labour for patients presenting toxemia of pregnancy, requires a relatively short t and a relatively high release rate (especially a SAP, such as $PGE_2$, which is hypotensive).

A device of the invention provides a rate-controlled, essentially time-independent release rate during treatment. The product of t and the release rate gives the therapeutic amount of the SAP delivered by the device.

Polymers which constitute the inner and outer membranes of a device of the invention are selected firstly on their ability to solubilise the SAP and to permit diffusion of the SAP. Accordingly, any polymeric substance known for use in delivery devices for lipophilic drugs is suitable, examples being the polyurethanes, styrene-butadiene block copolymers, polyesters, polysiloxanes, polyvinyl chlorides, ethylene vinyl acetates and polyalkylenes. As is apparent by reference to the above list of suitable polymers, the range of substances is limited only by the ability of a particular polymer to solubilise and diffuse the SAP.

Secondly, the polymers preferably have substantially different diffusion coefficients. The diffusion coefficient $(D_2)$ of the outer membrane is preferably at least 10, and more preferably at least 100, times that $(D_1)$ of the drug-bearing membrane. However, for devices which are used promptly after manufacture, there is less preference for substantially different diffusion coefficients; the polymers may in fact be the same.

The diffusion coefficients and respective solubilities $(S_1, S_2)$ of the SAP in the respective membranes are determined by standard experimental means, e.g. a diffusion chamber separated into two cells by a polymeric membrane having a thickness T. One cell contains an inert medium in which the SAP is dissolved, and maintained at a constant concentration, and the other cell contains only the inert medium. The concentration of SAP in the second cell is then plotted as a function of time, the function being linear after an initial nonlinear phase. For the linear function, the slope and abscissa (time-axis) intercept or time lag (L) can be determined graphically; the diffusion coefficient (D) and SAP solubility (S) are determined from:

$$D=T^2/6L \text{ and } S=T. \text{ slope}/D$$

After use, the amount of SAP remaining in a device of the invention is insufficient to permit medicinal reuse. This follows the definition that $y \geqslant 0.58x$, i.e. the time $(t_{50})$ between first administration and an attempted second administration during which the release rate has been reduced to no more than half its original value, is less than 0.5t. For a more severe constraint on non-reusability, $y > 0.25x$.

A preselected value for x/y provides not only a maximum, but also a minimum, value for $t_{50}/t$. In order that there should be a proper constraint on $t_{50}/t$, x/y should be so low that $t_{50}/t$ has a value above the minimum for the preselected x/y value. The bounds of the $t_{50}/t$ ratio may be expressed as follows:

$$3(x-y)1n2/(x+2y)<t_{50}/t<(x-y)1n2/y$$

For example, when x/y is 0.75, $0.77<t_{50}/t<0.79$.

The support used in a device of the invention is physiologically inert. Materials known to irritate, react or interact with rectal or vaginal epithelial tissue should be avoided. The material of the support should exhibit a certain resiliency, such that the physical integrity of the medicated device is maintained, on insertion. Resilient materials preferably evidence some small degree of compressability or deformability such that facile administration is accomplished, but substantially rigid support means may optionally be employed. Finally, support means must be water-insoluble, such that during the course of administration the secretions associated with vaginal or rectal epitherial tissue do not compromise the structural integrity of the device.

Just as the inner surface of the rate-controlling membrane is laminated onto the outer surface of the drug-bearing membrane, the inner surface of the drug-bearing membrane is laminated onto the support means. Accordingly, the shape, dimensions, and contours of the device of the present invention are essentially those of the support means. Hence, the support means must be adapted, contoured and dimensioned practicably for accomplishing the purposes of the instant invention. Specifically, the support means must facilitate the easy and comfortable insertion and withdrawal of the device into or from the vagina or rectum.

The support means of the present invention also exhibits numerous miscellaneous properties, namely being non-absorptive of the SAP and being constructed such that essentially the entire surface area would be in complete and intimate contact with vaginal or rectal epithelial tissues and associated secretions during administration. Accordingly, useful support means in accordance with the present invention are those which are non-concave or at least substantially so.

In view of the foregoing, especially convenient and well adapted support means for devices in accordance with the present invention are catamenial tampons. Commercially available catamenial tampons ordinarily contain an acceptably-sized surface area, are non-absorptive of lipophilic substances, and are water-insoluble. Morevoer, catamenial tampons are speccfically adapted, contoured and dimensioned for accommodation in the vagina.

Typically, catamenial tampons contain in addition to the corpus thereof a withdrawing means, ordinarily, simply a string. Devices in accordance with the present invention likewise preferably include as a further element thereof a withdrawing means, preferably a string or string-like appendage non-removably attached to the device itself.

There are accordingly provided medicated devices in accordance with the present invention which are surprisingly and unexpectedly capable of time-independent release of drug during a predetermined therapeutic duration, while avoiding the possibility of reuse of such devices thereafter. Moreover, extended use of devices in accordance with the present invention beyond their intended therapeutic duration, results in a surprisingly and unexpectedly rapid reduction in release rate therefrom, whereby physiologically consequential dosages from such extended administrations are minimized, if not avoided. Thus, the coupling in devices in accordance with the present invention of a time-independent release rate with non-reusability provides a surprisingly and unexpectedly improved means for the induction of desirable theraeptuc effects, particularly discrete events in the mammalian reproductive cycle. Hence, such devices avoid the difficulties inherent in prior art time-independent release devices where reusability creates serious disposal problems and drug misuse problems.

The drawings provide three views of a medicated device in accordance with the present invention, which are not drawn to scale, but rather to reflect the construction and operation of devices in accordance with the invention.

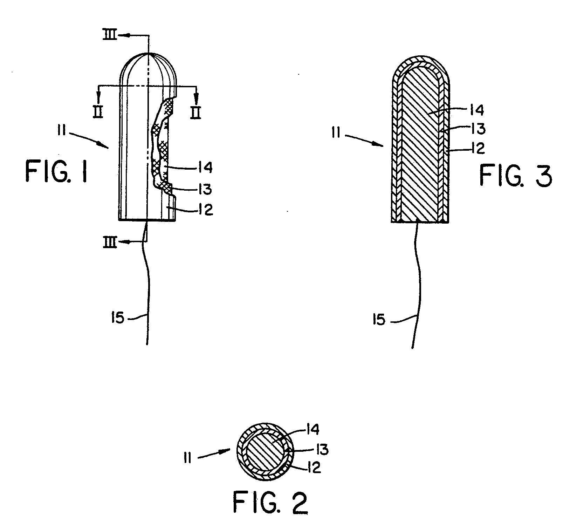

Figure 1 depicts the device, further providing a cut-away view of the interior therof, and depicts an embodiment exhibiting the optional withdrawing means.

Figures 2 and 3 are respectively transverse and longitudinal cross-sectional views of the device of Figure 1, respectively along the lines II—II and III—III.

The drawings show a medicated device whose outer surface is indicated generally at 11. An outer, rate-controlling membrane 12 is laminated to a substantially co-extensive, inner, rate-controlling membrane 13 which itself is laminated to a support 14 to which a withdrawing means 15 is non-removably attached.

In a general example of the preparation of a device of the invention, a commercially-available, compressed cotton catamenial tampon and polyurethanes are selected for use as the support and membrane materials. The polyurethanes are analysed in a two-cell diffusion chamber until two polyurethanes of substantially differing (i.e. 10—100 fold) diffusion coefficients are obtained. The membranes are prepared for lamination onto the tampon by a dip-coating method, employing an inverted test tube for membrane casting. Accordingly, a test tube is selected whose diameter is equal to or slightly less than that of the catamenial tampon, e.g. 5 mil. (0.13 mm). Firstly, the membrane having the higher coefficient is cast onto the test tube by dipping into a solution of water-miscible organic solvent, e.g. tetrahydrofuran, to which polymer (3—20% by weight) and SAP (0.5—10% by weight) have been added. The test tube, e.g. 5 mil (0.13 mm), is inserted and slowly withdrawn from the solution. The withdrawal rate is adjusted to obtain the desired thickness of the inner membrane. After drying and trimming, a second dip-coating is undertaken, employing a solution of the polymer selected for the outer membrane.

The laminated polymeric membranes are removed from the test tube by soaking in water (1—2 min.) and fixed to the catamenial tampon, e.g. by string tied at the base of the catamenial tampon (i.e. the juncture of the string-withdrawing means to the tampon).

This procedure may be used to obtain selected concentrations of SAP and thicknesses experimentally determined from solubilities, diffusion coefficients, x/y and t. Concentration and thickness values may be adjusted by amounts of no more than 10%, until devices of the desired performance are obtained, by methods obvious to those skilled in the art. For example, the thickness of the outer membrane may be reduced in order to increase an inadequate release rate with respect to area.

The following Example illustrate the invention.

Example

Following the general procedure outlined above, a device is constructed. The inner membrane consists of a layer of Estane 5714, a polyether-based urethane manufactured by B. F. Goodrich Company. The outer membrane is Elvax 40, an ethylene-vinyl acetate copolymer manufactured by E. I. DuPont Company, Inc. Each layer is 4 mil (0.1 mm) thick. The release area is 10 cm². The device contains 16 mg of $PGF_{2\alpha}$.

## Claims

1. A medicated device, adapted for use as a rectal or vaginal insert, having a surface area (A) of 10 to 50 cm², which comprises an inert support, an inner flexible polymeric membrane of substantially uniform thickness ($T_1$) laminated to the support and an outer flexible polymeric membrane of substantially uniform thickness ($T_2$) laminated to the inner membrane, in which the inner membrane contains, dissolved therein, an amount (x) of a systemically active pharmaceutical, characterised in that the device can release an amount (y) of the pharmaceutical at a constant rate of 1 to 1000 µg/hr over a time (t) no more than 72 hours, when in contact with rectal or vaginal tissue having a non-limiting absorption rate, with respect to the release rate, for the pharmaceutical, and

$$T_2y \simeq D_2S_2At$$
$$2(x-y) \simeq 2AS_1T_1 + AT_2S_2$$
$$T_2S_2D_1 > 10T_1S_1D_2$$
$$y \geqslant 0.58x$$

wherein $D_1$ and $D_2$ are the respective diffusion coefficients of the inner and outer membranes with respect to the pharmaceutical, and $S_1$ and $S_2$ are the respective solubilities of the pharmaceutical in the inner and outer membranes.

2. A device according to claim 1, in which the pharmaceutical is a prostaglandin.

3. A device according to Claim 1, in which the pharmaceutical is 15-methyl-$PGF_{2\alpha}$, methyl ester.

## Patentansprüche

1. Eine Arzneistoffe enthaltende, zum Anwenden als Rektal- bzw. Vaginaleinsatz bestimmte Vorrichtung mit einem Oberflächenmaß (A) von 10 bis 50 cm², bestehend aus einer inerten Stütze, aus einer inneren, an die Stütze kaschierten biegsamen polymerischen Membrane wesentlich gleichmäßiger Stärge ($T_1$) und aus einer äußeren, an die innere Membrane kaschierten, biegsamen polymerischen Membrane wesentlich gleichmäßiger Stärke ($T_2$), wobei die innere Membrane eine Menge (x) eines systemisch wirkenden Pharmazeutikums darin gelöst enthält, dadurch gekennzeichnet, daß die Vorrichtung bei Kontakt mit Rektal- bzw. Vaginal-gewebe mit einer mit Bezug auf die Freigabegeschwindigkeit nicht beschränkenden Absorptionsgeschwindigkeit für das Pharmazeutikum eine Menge (y) des Pharmazeutikums mit einer konstanten Geschwindigkeit von 1 bis 1000 µg/h über eine Zeitspanne (t) freigeben kann, wobei

$$T_2y \simeq D_2S_2At$$
$$2(x-y) \simeq 2AS_1T_1 + AT_2S_2$$
$$T_2S_2D_1 > 10T_1S_1D_2$$
$$y \geqslant 0,58x$$

wobei $D_1$ und $D_2$ die entsprechenden Diffusionsbeiwerte der inneren und der äußeren Membrane mit Bezug auf das Pharmazeutikum und $S_1$ und $S_2$ die entsprechenden Löslichkeiten des Pharmazeutikums in der inneren und der äußeren Membrane darstellen.

2. Eine Vorrichtung entsprechend Anspruch 1, bei welcher das Pharmazeutikum ein Prostaglandin ist.

3. Eine Vorrichtung nach Anspruch 1, bei welcher das Pharmazeutikum 15-Methyl-$PGF_{2\alpha}$, Methylester ist.

## Revendications

1. Dispositif médicamenté, conçu pour une insertion rectale ou vaginale, présentant une aire de surface (A) de 10 à 50 cm², qui comprend un support inerte, une membrane polymère flexible intérieure d'épaisseur sensiblement uniforme ($T_1$) appliquée sur le support et une membrane polymère flexible extérieure d'épaisseur sensiblement uniforme ($T_2$) appliquée sur la membrane intérieure, dans lequel la membrane intérieure contient, en dissolution, une quantité (x) d'un produit pharmaceutique à action systémique, caractérisé en ce que le dispositif peut libérer une quantité (y) du produit pharmaceutique à un débit constant de 1 à 1000 µg/h pendant un temps (t) ne dépassant pas 72 heures, lorsqu'il est au contact d'un tissu rectal ou vaginal ayant un débit d'absorption non limitatif, par rapport au débit de libération, du produit pharmaceutique, et

$$T_2y \cong D_2S_2At$$
$$2(x-y) \cong 2AS_1T_1 + AT_2S_2$$
$$T_2S_2D_1 > 10T_1S_1D_2$$
$$y \cong 0,58x$$

où $D_1$ et $D_2$ sont les coefficients respectifs de diffusion des membranes intérieure et extérieure par rapport au produit pharmaceutique, et $S_1$ et $S_2$ sont les solubilités respectives du produit pharmaceutique dans les membranes intérieure et extérieure.

2. Dispositif selon la revendication 1, dans lequel le produit pharmaceutique est une prostaglandine.

3. Dispositif selon la revendication 1, dans lequel le produit pharmaceutique est l'ester méthylique de 15-méthyl-$PGF_{2\alpha}$.

FIG. I

FIG. 3

FIG. 2